# EUROPEAN PATENT APPLICATION

(11) **EP 3 539 949 A1**
(43) Date of publication of application: **18.09.2019**
(21) Application number: 19160234.1
(22) Date of filing: 01.03.2019
(51) Int. Cl.: C07D 209/46, C07D 403/12, C07D 487/22

(54) **THERMOSET 3-OXO-ISOINDOLINE DIPHTHALONITRILE POLYMERS, METHOD OF MANUFACTURE, AND USES THEREOF**

(30) Priority: 14.03.2018 EP 18161711
(71) Applicant: SABIC Global Technologies B.V., 4612 PX Bergen op Zoom (NL)
(72) Inventor: CHOWDHURY, RAJESH, 562125 Bangalore, Karnataka (IN); KONDA, SHIVAKUMAR, 562125 Bangalore, Karnataka (IN)
(74) Representative: Modiano, Micaela Nadia

(57) **Abstract**

A thermoset polymer product comprising 4,4'-(((3-oxo-isoindoline-1,1-diyl)bis(4,1-arylene))bis(oxy))dibenzonitrile units of formula (2) 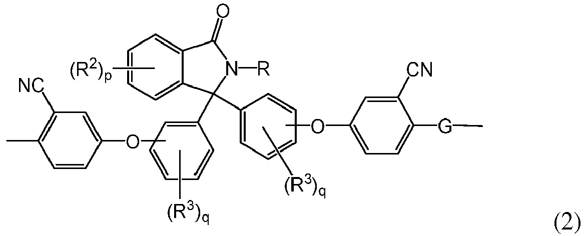 wherein the units are derived from a 4,4'-(((3-oxo-isoindoline-1,1-diyl)bis(4,1-arylene))bis(oxy))diphthalonitrile monomer of formula (1) 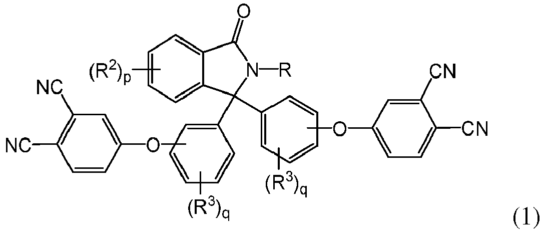 wherein R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl; p and q are each independently 0 to 4, preferably 0 or 1, more preferably 0; and G is a residue of a cyano group, and is a C₁₋₂₅ hydrocarbyl linking group having a valence of at least 2.

## Description

### BACKGROUND

Phthalonitrile polymers constitute an important class of high-temperature materials, having a wide range of uses, such as composite matrices, adhesives, sealants, and semiconductors. Phthalonitrile (PN) and diphthalonitrile (DPN) monomers can provide heat-curable crosslinked polymers having superior flammability and high temperature properties, relative to other high temperature polymers. The PN and DPN compounds can polymerize through the cyano groups to provide a crosslinked polymeric network with high thermal and oxidative stability, as well as a high modulus of elasticity.

DPN compounds include a spacer group that links the diphthalonitrile moieties. DPN compounds having aliphatic spacer groups are generally readily curable, compared to DPN compounds having aromatic spacer groups. For example, the cure temperature of DPN compounds having aromatic spacers can be higher than for aliphatic-spacer linked compounds. The relatively slow rate of polymerization for aromatic spacer-linked DPN compounds has been attributed to the rigidity of the linking structure, which can reduce the mobility of the cyano groups.

There accordingly remains a need in the art for thermoset polymer products derived from DPN monomers having aromatic bridging groups, and methods of synthesizing such thermoset polymers.

### SUMMARY

Described herein is a thermoset polymer product comprising 4,4'-(((3-oxo-isoindoline-1,1-diyl)bis(4,1-arylene))bis(oxy))dibenzonitrile units of formula (2) wherein the units are derived from a 4,4'-(((3-oxo-isoindoline-1,1-diyl)bis(4,1-arylene))bis(oxy))diphthalonitrile monomer of formula (1) 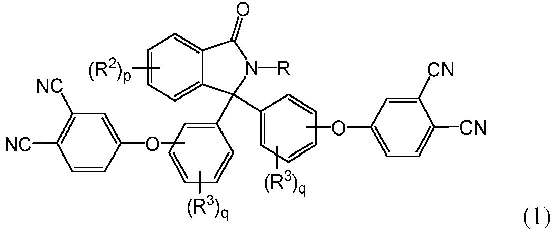 wherein R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl; p and q are each independently 0 to 4, preferably 0 or 1, more preferably 0; and G is a residue of a cyano group, and is a C₁₋₂₅ hydrocarbyl linking group having a valence of at least 2.

A polymer composition comprises the thermoset polymer product and an additive, preferably wherein the additive is a particulate filler, a fibrous filler, an antioxidant, a heat stabilizer, a light stabilizer, a ultraviolet light stabilizer, a ultraviolet light-absorbing compound, a near infrared light-absorbing compound, a infrared light-absorbing compound, a plasticizer, a lubricant, a release agent, a antistatic agent, an anti-fog agent, a antimicrobial agent, a colorant, a surface effect additive, a radiation stabilizer, a flame retardant, an anti-drip agent, a fragrance, a polymer different from the thermoset polymer, or a combination thereof.

Also disclosed is an article comprising the polymer composition, wherein the article is a composite, a foam, a fiber, a layer, a coating, an encapsulant, an adhesive, a sealant, a component, a prepreg, a casing, or a combination thereof; preferably wherein the article is an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof.

A method for the manufacture of the thermoset polymer product comprises polymerizing a 4,4'-(((3-oxo-isoindoline-1,1-diyl)bis(4,1-arylene))bis(oxy))diphthalonitrile monomer of formula (1) 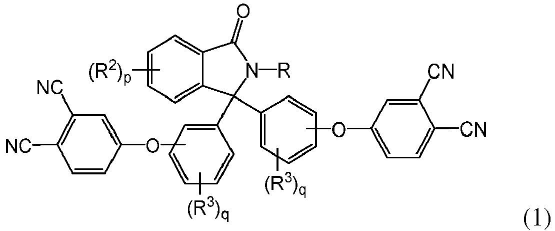 under conditions effective to form the thermoset polymer, wherein R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or phenyl; each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl; and p and q are each independently 0 to 4, preferably 0 or 1, more preferably 0.

The disclosure is further illustrated by the following detailed description, examples, and claims.

### DETAILED DESCRIPTION

Described herein are cured thermoset polymer products derived from compositions including diphthalonitrile (DPN) monomers having aromatic spacers that can have good thermo-oxidative resistance. The cured thermoset polymer products are made from 4,4'-(((3-oxo-isoindoline-1,1-diyl)bis(4,1-arylene))bis(oxy))diphthalonitrile monomers with potentially well-defined melting points. Such aromatic spacer-linked DPN monomers can provide thermoset polymers that can withstand high temperatures for extended periods of time without permanent damage. In particular, the thermoset polymers can withstand temperatures of up 500°C, preferably 200 to 500°C, more preferably 200 to 400°C. The thermoset polymers derived from the aromatic DPN monomers can be superior in physical and chemical properties to epoxies, polyimides, and other polymers used as high temperature adhesives.

Disclosed herein are thermoset polymer products of the DPN monomer, 4,4'-(((3-oxo-isoindoline-1,1-diyl)bis(4,1-arylene))bis(oxy))diphthalonitrile, having formula (1) 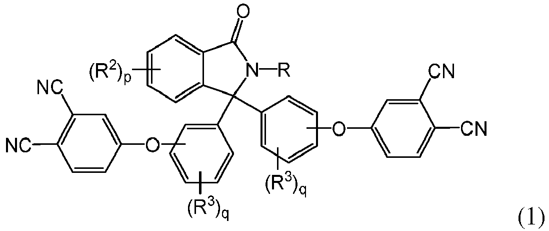 wherein R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl. Each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl. In formula (1), p and q are each independently 0 to 4, preferably 0 or 1, more preferably 0. For example, R is a C₁₋₃ alkyl or a phenyl, each occurrence of R² and R³ is independently a hydrogen, a halogen, or a C₁₋₆ alkyl, and p and q are each independently 0 or 1. In another example, R is a C₁₋₃ alkyl or a phenyl, each occurrence of R² and R³ is independently a hydrogen or a C₁₋₃ alkyl, and p and q are each 0.

The DPN monomer can be a 4,4'-(((3-oxo-2-arylisoindoline-1,1-diyl)bis(4,1-phenylene))bis(oxy))diphthalonitrile monomer of formula (1a) 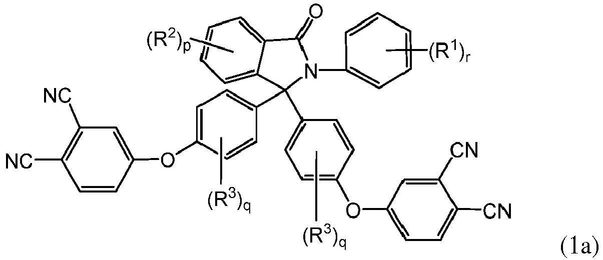 wherein each occurrence of R¹ is independently a hydrogen, a phenyl, or a C₁₋₆ alkyl, preferably a hydrogen or a C₁₋₃ alkyl. Each occurrence of R² and R³ is independently a hydrogen or a C₁₋₆ alkyl, preferably a hydrogen or a C₁₋₃ alkyl. In formula (1a), r, p, and q are each independently 0 or 1, preferably 0. For example, each occurrence of R¹ is independently a hydrogen or a C₁₋₃ alkyl, each occurrence of R² and R³ is independently a hydrogen or a C₁₋₃ alkyl, and r, p, and q are each independently 0 or 1, preferably 0.

In particular aspects, the DPN monomer can be a 4'-(((3-oxo-2-phenyisoindoline-1,1-diyl)bis(4,1-phenylene))bis(oxy))diphthalonitrile of formula (1b)

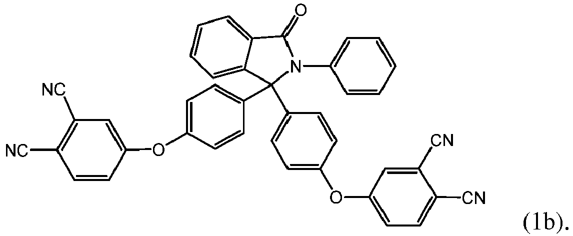

The thermoset product of the DPN monomer includes 4,4'-(((3-oxo-isoindoline-1,1-diyl)bis(4,1-arylene))bis(oxy))dibenzonitrile units of formula (2) 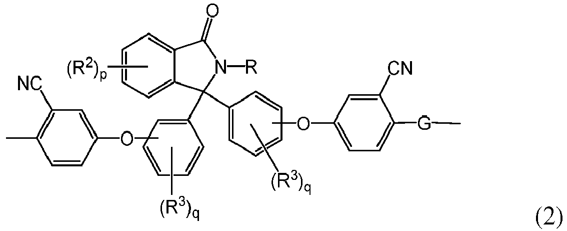 wherein R is a C₁₋₂₅ hydrocarbyl, preferably a C₁₋₆ alkyl, phenyl, or phenyl substituted with up to five C₁₋₆ alkyl groups, more preferably a C₁₋₃ alkyl or phenyl. Each occurrence of R² and R³ is independently a hydrogen, halogen, or C₁₋₂₅ hydrocarbyl, preferably a hydrogen, halogen, or C₁₋₆ alkyl, more preferably a hydrogen or C₁₋₃ alkyl. The group G is a residue of a cyano group, and is a C₁₋₂₅ hydrocarbyl linking group having a valence of at least 2. In formula (2), p and q are each independently 0 to 4, preferably 0 or 1, more preferably 0. For example, R is a C₁₋₃ alkyl or a phenyl, each occurrence of R² and R³ is independently a hydrogen, a halogen, or a C₁₋₆ alkyl, and P and q are each independently 0 or 1. In another aspect, R is a C₁₋₃ alkyl or a phenyl, each occurrence of R² and R³ is independently a hydrogen or a C₁₋₃ alkyl, and p and q are each 0.

In particular aspects, the thermoset polymer product has 4,4'-(((3-oxo-2-arylisoindoline-1,1-diyl)bis(4,1-phenylene))bis(oxy))dibenzonitrile units of formula (2a) 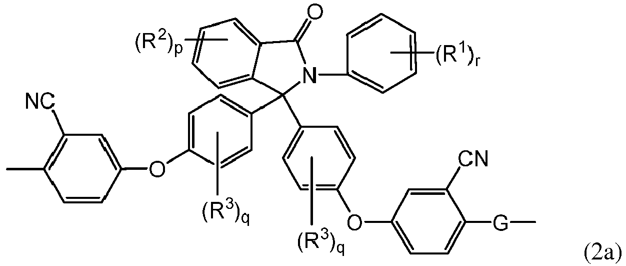 wherein each occurrence of R¹ is independently a hydrogen, phenyl, or C₁₋₆ alkyl, preferably a hydrogen or C₁₋₃ alkyl. Each occurrence of R² and R³ is independently a hydrogen or C₁₋₆ alkyl, preferably a hydrogen or C₁₋₃ alkyl. G is a residue of a nitrile group, wherein G is a C₁₋₆ hydrocarbyl linking group having a valence of 2-4. In formula (2a), r, p, and q are each independently 0 or 1, preferably 0. For example, each occurrence of R¹ is independently a hydrogen or a C₁₋₃ alkyl, each occurrence of R² and R³ is independently a hydrogen or a C₁₋₃ alkyl, and r, p, and q are each independently 0 or 1, preferably 0. For example, the thermoset polymer product can have 4,4'-(((3-oxo-2-phenylisoindoline-1,1-diyl)bis(4,1-phenylene))bis(oxy))dibenzonitrile units of formula (2b) 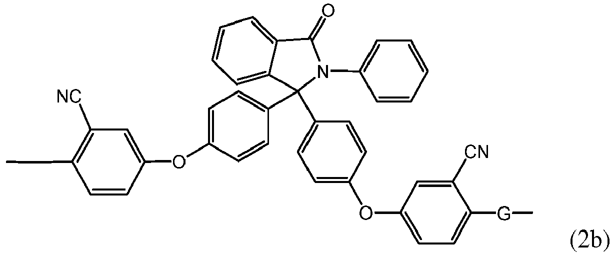 wherein G is a residue of a cyano group, and is a C₁₋₆ hydrocarbyl linking group having a valence of 2-4.

The residue of a cyano group G can be a phthalocyanine group or a triazinyl group. In a specific aspect, the cyano group G is a triazinyl group and the thermoset polymer product is of formula (2c)

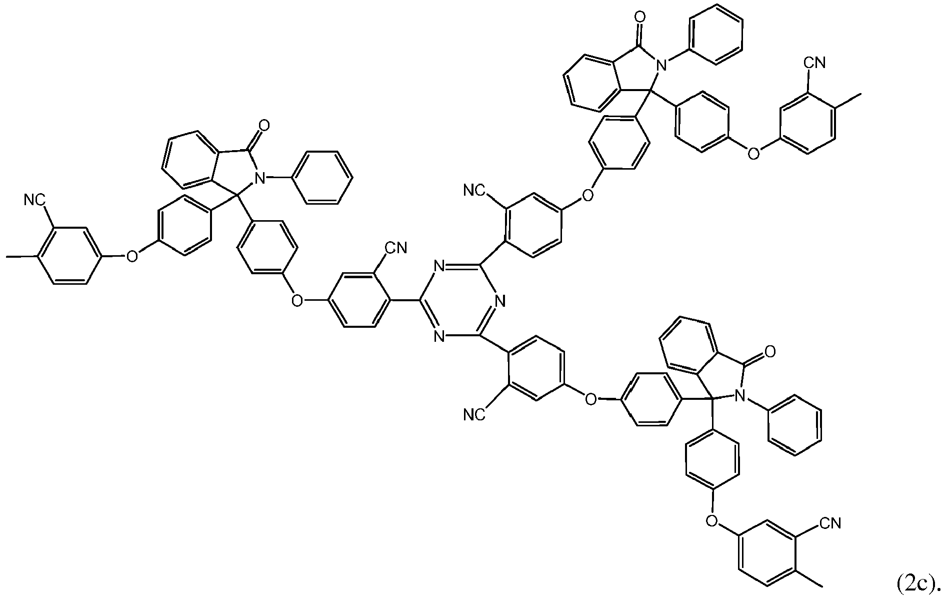

The thermoset polymer product can include cyclized polymers. For example, the thermoset polymer product can have 4,4'-(((3-oxo-isoindoline-1,1-diyl)bis(4,1-arylene))bis(oxy))dibenzo units of formula (3) 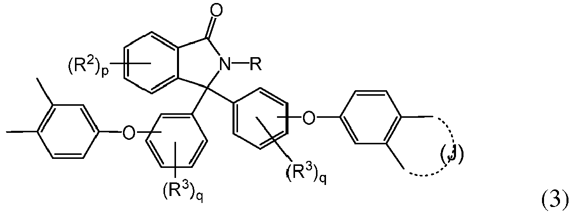 wherein R is a C₁₋₂₅ hydrocarbyl, preferably a C₁₋₆ alkyl, phenyl, or phenyl substituted with up to five C₁₋₆ alkyl groups, more preferably a C₁₋₃ alkyl or phenyl. Each occurrence of R² and R³ is independently a hydrogen, halogen, or C₁₋₂₅ hydrocarbyl, preferably a hydrogen, halogen, or C₁₋₆ alkyl, more preferably a hydrogen or C₁₋₃ alkyl. J is a residue of a cyano group, and is a C₁₋₂₅ hydrocarbyl linking group having a valence of at least 3. In formula (3), p and q are each independently 0 to 4, preferably 0 or 1, more preferably 0. For example, R is a C₁₋₃ alkyl or a phenyl, each occurrence of R² and R³ is independently a hydrogen, a halogen, or a C₁₋₆ alkyl, and p and q are each independently 0 or 1. In another aspect, R is a C₁₋₃ alkyl or a phenyl, each occurrence of R² and R³ is independently a hydrogen or a C₁₋₃ alkyl, and p and q are each 0.

The thermoset polymer product can have 4,4'-(((3-oxo-2-arylisoindoline-1,1-diyl)bis(4,1-phenylene))bis(oxy))dibenzo units of formula (3a) 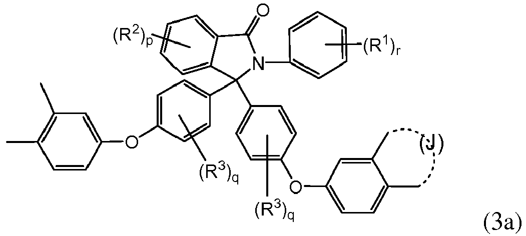 wherein each occurrence of R¹ is independently a hydrogen, phenyl, or C₁₋₆ alkyl, preferably a hydrogen or C₁₋₃ alkyl. Each occurrence of R² and R³ is independently a hydrogen or C₁₋₆ alkyl, preferably a hydrogen or C₁₋₃ alkyl. J is a residue of two nitrile groups, wherein J is a C₄₋₁₀ hydrocarbyl linking group having a valence of 2 to 4. In formula (3a), r, p, and q are each independently 0 or 1, preferably 0. For example, each occurrence of R¹ is independently a hydrogen or a C₁₋₃ alkyl, each occurrence of R² and R³ is independently a hydrogen or a C₁₋₃ alkyl, and r, p, and q are each independently 0 or 1, preferably 0.

The thermoset polymer product can have 4,4'-(((3-oxo-2-phenylisoindoline-1,1-diyl)bis(4,1-phenylene))bis(oxy))dibenzonitrile units of formula (3b) 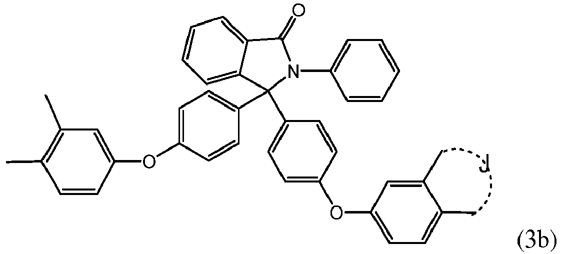 wherein J is a C₄₋₁₀ hydrocarbyl linking group having a valence of 2-4.

In a particular aspect, the thermoset polymer product is of formula (3c)

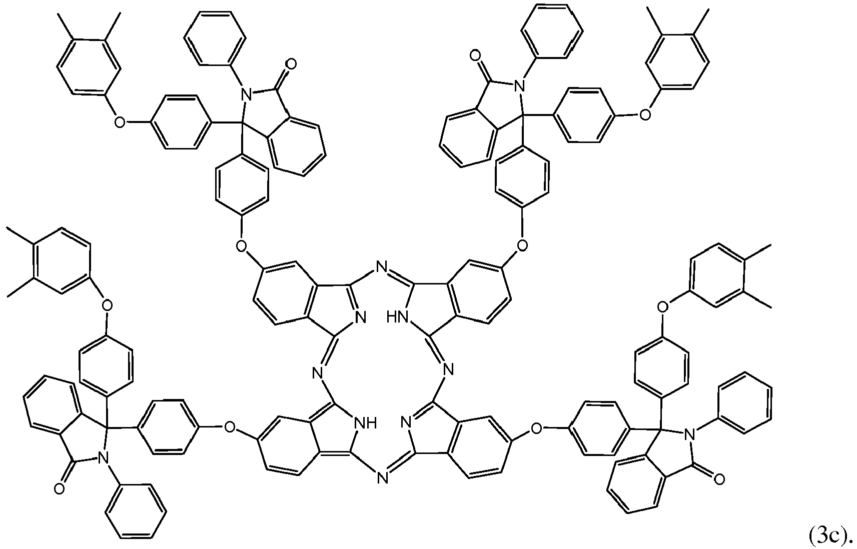

Also provided is a method for the manufacture of the thermoset polymer product described herein. The method can include polymerizing a 4,4'-(((3-oxo-isoindoline-1,1-diyl)bis(4,1-arylene))bis(oxy))diphthalonitrile monomer of formula (1), formula (1a), formula (1b), or a combination thereof, under conditions effective to form the thermoset polymer.

The method of polymerizing includes heating the thermosetting compound. The heating can be at a temperature of 200 to 500°C, preferably 200 to 400°C, more preferably 200 to 300°C, even more preferably 220 to 300°C, optionally in the presence of a curing agent. The heating can further be at a pressure of 1 to 100 atmospheres (atm) (101.3 to 10132.5 kilopascals (kPa)), preferably 1 to 10 atm (101.3 to 1013.3 kPa), more preferably 1 to 5 atm (101.3 to 506.7 kPa). For example, the heating can be at a temperature of 200 to 500°C, preferably 200 to 400°C, more preferably 200 to 300°C, even more preferably 220 to 300°C and at a pressure of 1 to 100 atm (101.3 to 10132.5 kPa), preferably 1 to 10 atm (101.3 to 1013.3 kPa), more preferably 1 to 5 atm (101.3 to 506.7 kPa).

The heating can comprise a cure cycle. For example, the cure cycle can include a two-part cure of 225 to 260°C for 5 to 20 hours and 300 to 315°C for 5 to 20 hours; a three-part cure of 180 to 240°C for 2 to 16 hours, 240 to 300°C for 2 to 8 hours, and 300 to 315°C for 4 to 20 hours; or a four-part cure of 200 to 240°C for 1 to 3 hours, 240 to 270°C for 2 to 4 hours, 270 to 300°C for 4 to 6 hours, and 300 to 315°C for 4 to 20 hours.

The diphthalonitrile monomers are generally solids at room or ambient temperatures, and can have melting points between 200 to 300°C. The polymerization of the diphthalonitrile monomers can be sluggish and can require heating at elevated temperature for extended time before a vitrified product is obtained. Therefore, a curing agent can be used to promote the thermal polymerization reaction of the diphthalonitrile monomers. Exemplary curing agents include, but are not limited to, organic amines, phenols, strong organic acids (e.g., organic acids having an acid dissociation constant (pKₐ) in water of less than 4), and amine salts thereof, metals and salts thereof, or a combination thereof.

Preferably the curing agent is an amine-containing phosphazene, an aromatic diamine, an aromatic diamine/metal salt complex, an aromatic ether amine, an amine salt of p-toluene sulfonic acid, or an organic acid having a pKₐ in water of less than 3. More preferably the curing agent can be an aromatic diamine or an organic acid having a pKₐ in water of less than 2. A combination of curing agents can also be used.

For example, the curing agent can be an organic acid or combination of organic acids, exhibiting a pKₐ in water of less than 1.0. More preferably, a strong organic acid exhibits a pKₐ in water of less than 0.80. Strong organic acids include, for example, aromatic acids containing inorganic acidic substituents, such as the sulfonic group -SO₃H. Exemplary organic aromatic acids include p-toluenesulfonic acid, aniline-2-sulfonic acid, 8-aniline-1-naphthalenesulfonic acid, benzene sulfonic acid, butylsulfonic acid, 10-camphorsulfonic acid,2,5-diaminobenzenesulfonicacid,6-dimethylamino-4-hydroxy-2-naphthalenesulfonic acid, 5-dimethylamino-1-naphthalenesulfonic acid, 4-hydroxy-3-nitroso-1-naphthalenesulfonic acid tetrahydrate, 8-hydroxyquinoline-5-sulfonic acid, methylsulfonic acid, phenylboric acid, 1-naphthalenesulfonic acid, 2-naphthalenesulfonic acid, 1,5- naphthalenedisulfonic acid, 2,6-naphthalenedisulfonic acid, 2,7- napththalenedisulfonic acid, picrylsulfonic acid hydrate, 2-pyridineethanesulfonic acid, 4-pyridineethanesulfonic acid, 3-pyridinesulfonic acid, 2-pyridinylhydroxymethanesulfonic acid, sulfanilic acid, 2-sulfobenzoic acid hydrate, 5-sulfosalicylic acid hydrate, 2,4-xylenesulfonic acid, sulfonic acid containing dyes, or a combination thereof.

The curing agent can be an aromatic diamine, such as a substituted or unsubstituted aromatic diamine of the formula (4)

Z(NH₂)₂ (4)

wherein Z is a C₆₋₂₂ arylene or a substituted derivative thereof. For example, the aromatic diamine can be o-phenylenediamine, m-phenylenediamine, p-phenylenediamine, 4,4'-diaminodiphenylpropane, 4,4'-diaminodiphenylmethane, 4,4'-diaminodiphenyl sulfide, 4,4'-diaminodiphenyl ether, 1,5-diaminonaphthalene, 3,3'-dimethylbenzidine, 3,3'-dimethoxybenzidine, 2,4-bis(β-amino-t-butyl)toluene, bis(p-β-amino-t-butyl)ether, bis(p-β-methyl-o-aminopentyl)benzene, 1,3-diamino-4-isopropylbenzene, 1,2-bis(3-aminopropoxy)ethane, benzidine, m-xylylenediamine, p-xylylenediamine, 2,4-diaminotoluene, 2,6-diaminotoluene, 1,3-bis(3-aminophenoxy)benzene, 1,3-bis(4-aminophenoxy)benzene, 1,4-bis(3-aminophenoxy)benzene, 1,4-bis(4-aminophenoxy)benzene, bis[4-(3-aminophenoxy)phenyl]sulfone, bis[4-(4-aminophenoxy)phenyl]sulfone, 4,4'-bis(3-aminophenoxy)biphenyl, 4,4'-bis(4-aminophenoxy)biphenyl, 2,2-bis[4-(3-aminophenoxy)phenyl]propane, 2,2-bis[4-(4-aminophenoxy)phenyl]propane, or a combination thereof.

The curing agent can be a salt of any of the amines disclosed herein. Exemplary curing salts include bis(3-aminophenoxy)-1,3-benzene p-toluene sulfonate, p-phenylenediamine p-toluenesulfonate, bis(4-aminophenyl)methane hydrochloride, N-phenylbenzamidine p-toluene sulfonate, or a combination thereof.

The curing agent can be metal salt complex of the diamines disclosed herein. Examples of metal salts include cuprous chloride, cuprous bromide, cuprous cyanide, cuprous ferricyanide, zinc chloride, zinc bromide, zinc iodide, zinc cyanide, zinc ferrocyanide, zinc acetate, zinc sulfide, silver chloride, ferrous chloride, ferric chloride, ferrous ferricyanide, ferrous chloroplatinate, ferrous fluoride, ferrous sulfate, cobaltous chloride, cobaltic sulfate, cobaltous cyanide, nickel chloride, nickel cyanide, nickel sulfate, nickel carbonate, stannic chloride, stannous chloride hydrate, a complex of triphenylphosphine oxide and stannous chloride, or a combination thereof.

The curing agent can be an aromatic ether amine. Aromatic ether amines include those described in U.S. Pat. No. 5,003,078, the entire content of which is incorporated herein by reference. Exemplary aromatic ether amines include 1,3-bis(3-aminophenoxy)benzene, 1,3-bis(4-aminophenoxy)benzene, 1,4-bis(3-aminophenoxy)benzene, 1,4-bis(4-aminophenoxy)benzene, 4,4'-bis(3-aminophenoxy)biphenyl, 4,4'-bis(4-aminophenoxy)biphenyl, 2,2-bis[4-(3-aminophenoxy)phenyl]propane, 2,2-bis[4-(4-aminophenoxy)phenyl]propane, or a combination thereof.

The curing agent can be an amine-containing phosphazene. Amine-containing phosphazenes include those described in U.S. Pat. No. 5,208,318, the entire content of which is incorporated herein by reference.

An effective amount of the curing agent can be used. For example, the amount by weight of the curing agent can be 1 to 40 wt%, preferably 1 to 20 wt%, more preferably 5 to 10 wt%, based on the weight of the monomer. In a particular example, the curing agent is an amine and the amount of curing agent is 1 to 20 wt%, preferably 1 to 15 wt%, more preferably 5 to 10 wt%, based on the weight of the monomer.

The 4,4'-(((3-oxo-isoindoline-1,1-diyl)bis(4,1-arylene))bis(oxy))diphthalonitrile monomers of formula (1) can be prepared by the reaction of 4-nitrophthalonitrile and a bisphenol of formula (5) 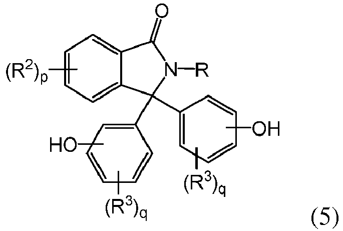 under conditions effective to provide 4,4'-(((3-oxo-isoindoline-1,1-diyl)bis(4,1-arylene))bis(oxy))diphthalonitrile monomer of formula (1). The effective conditions can include stirring at 10°C to 50°C for 6 to 48 hours, preferably at 15°C to 40°C for 12 to 36 hours. The molar ratio of the bisphenol of formula (5) to the 4-nitrophthalonitrile can be 1:1 to 1:5, preferably 1:1 to 1:3.

The monomers can be prepared in a solvent and in the presence of an inorganic base. Exemplary solvents can include an alkyl pyrrolidone solvent, an aromatic solvent, a halogenated aromatic solvent, or combination thereof. For example, the solvent comprises N-methyl-2-pyrrolidone, toluene, xylene, ortho-dichlorobenzene, 1,3-dimethyl-2-imidazolinone, 2-pyrrolidone, or a combination thereof. Exemplary inorganic bases include alkali metal and alkaline earth metal salts of carbonate, sulfate, and phosphate. In some aspects, the inorganic base is lithium carbonate, sodium carbonate, potassium carbonate, cesium carbonate, magnesium carbonate, calcium carbonate, or a combination thereof. In a particular example, the base is potassium carbonate.

In a particular aspect, the monomers are prepared by contacting an inorganic base and a 3,3-bis(4-hydroxyaryl)-2-arylisoindolin-1-one of formula (5) to provide a metal salt precursor, and reacting the metal salt precursor with 4-nitrophthalonitrile under conditions effective to provide the 4,4'-(((3-oxo-isoindoline-1,1-diyl)bis(4,1-arylene))bis(oxy))diphthalonitrile compound of formula (1).

The monomers can be prepared in the presence of a phase transfer catalyst. Examples of phase transfer catalysts include quaternary ammonium salts, quaternary phosphonium salts, sulphoxides, sulpholanes, guanidinium salts, imidazolium salts, or a combination thereof. Exemplary catalysts include tetramethylammonium chloride, tetramethylammonium bromide, trimethylbenzylammonium chloride, triethylbenzylammonium chloride, cetyltrimethylammonium bromide, methyltrioctylammonium chloride, trioctylbenzylammonium chloride, tributylbenzylammonium chloride , tetrabutylammonium bromide, tetraphenylphosphonium halides, methyltriphenylphosphonium iodide, ethyltriphenylphoshonium chloride, dimethylsulfoxide, diphenylsulfoxide, dibutylsulfoxide, sulfolane, 2,4-dimethylsulfolane, dimethylsulfone, diphenylsulfone, hexaethylguanidinium chloride, 1-butyl-3-methylimidazolium chloride, or a combination thereof. The phase transfer catalyst can be present in an amount of 0.5 to 80 mol%, based on the moles of bisphenol (5).

Also provided is a polymer composition comprising the thermoset polymer product disclosed herein and an additive. The additive can be a particulate filler, a fibrous filler, an antioxidant, a heat stabilizer, a light stabilizer, a ultraviolet light stabilizer, a ultraviolet light-absorbing compound, a near infrared light-absorbing compound, a infrared light-absorbing compound, a plasticizer, a lubricant, a release agent, a antistatic agent, an anti-fog agent, a antimicrobial agent, a colorant, a surface effect additive, a radiation stabilizer, a flame retardant, an anti-drip agent, a fragrance, a polymer different from the thermoset polymer, or a combination thereof.

Examples of polymers different than the thermoset polymer include polyacetals (e.g., polyoxyethylene and polyoxymethylene), poly(C₁₋₆ alkyl)acrylates, polyacrylamides, polyamides, (e.g., aliphatic polyamides, polyphthalamides, and polyaramides), polyamideimides, polyanhydrides, polyarylates, polyarylene ethers (e.g., polyphenylene ethers), polyarylene sulfides (e.g., polyphenylene sulfides), polyarylene sulfones (e.g., polyphenylene sulfones), polybenzothiazoles, polybenzoxazoles, polycarbonates (including polycarbonate copolymers such as polycarbonate-siloxanes, polycarbonate-esters, and polycarbonate-ester-siloxanes), polyesters (e.g., polyethylene terephthalates, polybutylene terephthalates, polyarylates, and polyester copolymers such as polyester-ethers), polyetheretherketones, polyetherimides (including copolymers such as polyetherimide-siloxane copolymers), polyetherketoneketones, polyetherketones, polyethersulfones, polyimides (including copolymers such as polyimide-siloxane copolymers), poly(C₁₋₆ alkyl)methacrylates, polymethacrylamides, polynorbornenes (including copolymers containing norbornenyl units), polyolefins (e.g., polyethylenes, polypropylenes, polytetrafluoroethylenes, and their copolymers, for example ethylene-alphaolefin copolymers), polyoxadiazoles, polyoxymethylenes, polyphthalides, polysilazanes, polysiloxanes, polystyrenes (including copolymers such as acrylonitrile-butadiene-styrene (ABS) and methyl methacrylate-butadiene-styrene (MBS)), polysulfides, polysulfonamides, polysulfonates, polysulfones, polythioesters, polytriazines, polyureas, polyurethanes, polyvinyl alcohols, polyvinyl esters, polyvinyl ethers, polyvinyl halides, polyvinyl ketones, polyvinyl thioethers, polyvinylidene fluorides, or the like, or a combination thereof.

Also provided is an article including the polymer composition. The article can be in the form of a composite, a foam, a fiber, a layer, a coating, an encapsulant, an adhesive, a sealant, a component, a prepreg, a casing, or a combination thereof. Specific examples of the articles include an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof. Additional exemplary articles include, for example, a carbon fiber composite, a fiberglass composite, a fiber coating, a weather resistant coating, a scratch resistant coating, a food packaging coating, a electrical insulative coating, a carbon fiber composite, a fiberglass composite, a industrial component, a oil rig component, a missile component, a rocket component, a building component, a bridge component, a transportation component, a electrical component, a computer component, a appliance component, a furniture component, a pump component, a electrolytic cell component, a tooling, a flame retardant component, a body protective component, a sporting equipment component, a container, a filament winding, a resin infusion molding component, a resin transfer molding component, or a combination thereof.

For example, the article can be an exhaust stack, a scrubber, a stair case, a walkway, a tile, a vehicle, a aircraft or watercraft exterior component, submersible watercraft component, a floor pan, an air scoop, a pipe, a duct, a fan component, a fan housing, a blower, a industrial mixer component, a tile, a business machine component, or a pultruded part for structural applications. Other exemplary articles include a water craft hull, a watercraft deck, a marine terminal fender, a bridge deck, a bridge support, a railing, a building panel, a business machine housing or tray, a concrete modifier, a dishwasher or refrigerator part, an electrical encapsulant, a electrical panel, a electrorefining tank, a water softener tank, a fuel tank, a pressure tank, a filament-wound tank, a tank lining, a garage door, a grating, a protective body gear, a luggage component, a printed circuit board, an optical waveguide, a radome, a tank car, a hopper car cover, a car door, a truck bed liner, a satellite dish, a sign, a solar energy panel, a telephone switchgear housing, a tractor part, a transformer cover, a ground insulation, a turn insulation, a phase separation insulation for rotating machines, a commutator, a core insulation, a cord, a lacing tape, a drive shaft coupling, a propeller blade, a rocket motor case, a wing section, a sucker rod, a fuselage section, a wing skin wing fairing, a engine narcelle, a cargo door, a tennis racquet, a golf club shaft, a fishing rod, a ski, a ski pole, a bicycle part, a transverse leaf spring, a automotive smog pump component, a such as electrical cable joint, a wire winding, a sealant for an electromechanical device, a battery case, a resistor, a fuse, a thermal cut-off device, a capacitor, a transformer, a crankcase heater, a coil, a resistor, a semiconductor, a steel replacement component in chemical processing, a pulp and paper, a power generation, a and wastewater treatment, a scrubbing tower, a gratings, a safety rail, a component for a swimming pool, a swimming pool slide, a hot-tub, a or sauna, a drive shaft, a dry toner polymer for copying machine, a heat shield, a submarine hull, a laminated trim, a drilling fixture, a bonding jig, a inspection fixture, a industrial metal forming die, a aircraft stretch block and hammer form, a vacuum molding tool, a flooring, a antistatic articles, a decorative flooring; expansion joints for bridge, an injectable mortar for patch and repair of cracks in structural concrete, a grouting for tile, a machinery rail, a bolt and post, a storage tank repair, or a light emitting diode (LED) encapsulant.

Processes useful for preparing the articles and materials include those generally known to the art for the processing of thermosetting polymers. Such processes have been described in the literature as in, for example, Engineered Materials Handbook, Volume 1, Composites, ASM International Metals Park, Ohio, copyright 1987 Cyril A. Dostal Senior Ed, pp. 105-168 and 497-533, and "Polyesters and Their Applications" by Bjorksten Research Laboratories, Johan Bjorksten (pres.) Henry Tovey (Ch. Lit. Ass.), Betty Harker (Ad. Ass.), James Henning (Ad. Ass.), Reinhold Publishing Corporation, New York, 1956. Processing techniques include polymer transfer molding; sheet molding; bulk molding; pultrusion; injection molding, including reaction injection molding (RIM); atmospheric pressure molding (APM); casting, including centrifugal and static casting open mold casting; lamination including wet or dry lay up and spray lay up; also included are contact molding, including cylindrical contact molding; compression molding; including vacuum assisted polymer transfer molding and chemically assisted polymer transfer molding; matched tool molding; autoclave curing; thermal curing in air; vacuum bagging; pultrusion; Seeman's Composite Resin Infusion Manufacturing Processing (SCRIMP); open molding, continuous combination of polymer and glass; and filament winding, including cylindrical filament winding. The article can be prepared from the disclosed thermoset polymer product via a polymer transfer molding process.

The compositions and methods described herein are further illustrated by the following non-limiting examples.

### EXAMPLES

The materials listed in Table 1 were used.

**Table 1.**

| Component | Description | Source |
|---|---|---|
| ODA | 4,4'-Oxydianiline | Sigma-Aldrich |
| Diphthalonitrile monomer | 4,4'-(((3-oxo-2-phenylisoindoline-1,1-diyl)bis(4,1-phenylene))bis(oxy))diphthalonitrile monomer | SABIC |

### Example 1. Crosslinking of 4,4'-(((3-oxo-2-phenylisoindoline-1,1-diyl)bis(4,1-phenylene))bis(oxy))diphthalonitrile monomer (1b).

The aromatic diamine 4,4'-oxydianiline (ODA) was used as curing agent to promote the thermal cure reaction of the diphthalonitrile monomer 4,4'-(((3-oxo-2-phenylisoindoline-1,1-diyl)bis(4,1-phenylene))bis(oxy))diphthalonitrile (1b) in in two stages. The first stage is addition of ODA to a melt of the diphthalonitrile monomer (1b) at 250°C, followed by quenching the reaction after 15 minutes to form diphthalonitrile prepolymer resin. The second stage is cure of the prepolymer using ODA.

The prepolymer synthesis was accomplished by melting 5.21 grams (g) (8.07 millimoles (mmol)) of the diphthalonitrile monomer (1b) in a 250-mL round bottomed flask at 250°C, adding 0.033 g (0.165 mmol) of the ODA, and stirring the mixture for 10 to 15 minutes in ambient air followed by quenching the a dark green prepolymer melt composition to 23°C. The prepolymer was finely ground and used for curing. The samples were cured by heating at 270°C for 5 hours, 300°C for 5 hours, 340°C for 5 hours, and 400°C for 5 hours under inert conditions (e.g., N₂ or Ar).

The characteristic nitrile absorption band was observed at 2234 cm⁻¹ in the Fourier transform infrared (FTIR) spectra of the diphthalonitrile monomer and the resulting thermoset polymer. The intensity of the nitrile absorption of the thermoset was 15 to 25% of the original intensity of the diphthalonitrile monomer. The intensity of the absorption of C-N stretch in the range of 1527 cm⁻¹ increased noticeably, indicating the formation of a triazine ring during the curing process. The emergence of a new peak having weak intensity at about 1010 cm⁻¹ was attributed to the formation of a phthalocyanine ring.

The glass transition temperature of the cured thermoset polymer was 390°C to 410°C, as measured using differential scanning calorimetry (DSC) at a temperature profile of 250°C to 600°C at a ramp rate of 10°C per minute.

Table 2 shows the results of thermogravimetric analysis (TGA) for the diphthalonitrile monomer (1b) and the thermoset polymer.

**Table 2.**

| Sample | T_{5%} (°C) | T_{10%} (°C) | % Residue at 800°C |
|---|---|---|---|
| Diphthalonitrile monomer in nitrogen | 362.8 | 389.8 | 49.64 |
| Diphthalonitrile polymer in nitrogen | 500.2 | 546.3 | 78.05 |
| Diphthalonitrile monomer in air | 367.1 | 397.6 | 17.36 |
| Diphthalonitrile polymer in air | 485.1 | 528.9 | 18.81 |

The TGA of the cured thermoset polymer confirmed the outstanding thermo-oxidative stability of the groups in the diphthalonitrile monomer. As shown above, the diphthalonitrile polymers had higher thermal decomposition temperatures at both 5% (T_{5%}) and 10% (T_{10%}) weight losses compared to the diphthalonitrile monomers.

The disclosure is further illustrated by the following non-limiting aspects.

Aspect 1. A thermoset polymer product comprising 4,4'-(((3-oxo-isoindoline-1,1-diyl)bis(4,1-arylene))bis(oxy))dibenzonitrile units of formula (2) wherein the units are derived from a 4,4'-(((3-oxo-isoindoline-1,1-diyl)bis(4,1-arylene))bis(oxy))diphthalonitrile monomer of formula (1), wherein R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl; p and q are each independently 0-4, preferably 0 or 1, more preferably 0; and G is a residue of a cyano group, and is a C₁₋₂₅ hydrocarbyl linking group having a valence of at least 2.

Aspect 2. The thermoset polymer product of aspect 1, wherein the monomer is a 4,4'-(((3-oxo-2-arylisoindoline-1,1-diyl)bis(4,1-phenylene))bis(oxy))diphthalonitrile monomer of formula (1a) wherein each occurrence of R1 is independently a hydrogen, a phenyl, or a C₁₋₆ alkyl, preferably a hydrogen or a C₁₋₃ alkyl; each occurrence of R² and R³ is independently a hydrogen or a C₁₋₆ alkyl, preferably a hydrogen or a C₁₋₃ alkyl; and r, p, and q are each independently 0 or 1, preferably 0.

Aspect 3. The thermoset polymer product of aspect 2, wherein the monomer is 4'-(((3-oxo-2-phenylisoindoline-1,1-diyl)bis(4,1-phenylene))bis(oxy))diphthalonitrile of formula (1b).

Aspect 4. The thermoset polymer product of any one or more of the preceding aspects, comprising 4,4'-(((3-oxo-2-arylisoindoline-1,1-diyl)bis(4,1-phenylene))bis(oxy))dibenzonitrile units of formula (2a) wherein each occurrence of R¹ is independently a hydrogen, phenyl, or C₁₋₆ alkyl, preferably a hydrogen or C₁₋₃ alkyl; each occurrence of R² and R³ is independently a hydrogen or C₁₋₆ alkyl, preferably a hydrogen or C₁₋₃ alkyl; r, p, and q are each independently 0 or 1, preferably 0; and G is a residue of a nitrile group, wherein G is a C₁₋₆ hydrocarbyl linking group having a valence of 2-4.

Aspect 5. The thermoset polymer product of any one or more of the preceding aspects, wherein G is a phthalocyanine group or a triazinyl group.

Aspect 6. The thermoset polymer product of any one or more of the preceding aspects, having the formula (2c).

Aspect 7. The thermoset polymer product of any one or more of the preceding aspects, comprising 4,4'-(((3-oxo-isoindoline-1,1-diyl)bis(4,1-arylene))bis(oxy))dibenzo units of formula (3) wherein R is a C₁₋₂₅ hydrocarbyl, preferably a C₁₋₆ alkyl, phenyl, or phenyl substituted with up to five C₁₋₆ alkyl groups, more preferably a C₁₋₃ alkyl or phenyl; each occurrence of R² and R³ is independently a hydrogen, halogen, or C₁₋₂₅ hydrocarbyl, preferably a hydrogen, halogen, or C₁₋₆ alkyl, more preferably a hydrogen or C₁₋₃ alkyl; and p and q are each independently 0-4, preferably 0 or 1, more preferably 0; and J is a residue of a cyano group, and is a C₁₋₂₅ hydrocarbyl linking group having a valence of at least 3.

Aspect 8. The thermoset polymer product of aspect 8, comprising 4,4'-(((3-oxo-2-arylisoindoline-1,1-diyl)bis(4,1-phenylene))bis(oxy))dibenzo units of formula (3a) wherein each occurrence of R¹ is independently a hydrogen, phenyl, or C₁₋₆ alkyl, preferably a hydrogen or C₁₋₃ alkyl; each occurrence of R² and R³ is independently a hydrogen or C₁₋₆ alkyl, preferably a hydrogen or C₁₋₃ alkyl; r, p, and q are each independently 0 or 1, preferably 0; and J is a residue of two nitrile groups, wherein J is a C₄₋₁₀ hydrocarbyl linking group having a valence of 2-4.

Aspect 9. The thermoset polymer product of aspect 7 or 8, having the formula (3c).

Aspect 10. A polymer composition comprising the thermoset polymer product of any one or more of the preceding aspects, and an additive, preferably wherein the additive is a particulate filler, a fibrous filler, an antioxidant, a heat stabilizer, a light stabilizer, a ultraviolet light stabilizer, a ultraviolet light-absorbing compound, a near infrared light-absorbing compound, a infrared light-absorbing compound, a plasticizer, a lubricant, a release agent, a antistatic agent, an anti-fog agent, a antimicrobial agent, a colorant, a surface effect additive, a radiation stabilizer, a flame retardant, an anti-drip agent, a fragrance, a polymer different from the thermoset polymer, or a combination thereof.

Aspect 11. An article comprising the polymer composition of aspect 10, wherein the article is a composite, a foam, a fiber, a layer, a coating, an encapsulant, an adhesive, a sealant, a component, a prepreg, a casing, or a combination thereof; preferably wherein the article is an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof.

Aspect 12. A method for the manufacture of the thermoset polymer product of any one or more of aspects 1 to 9, the method comprising polymerizing a 4,4'-(((3-oxo-isoindoline-1,1-diyl)bis(4,1-arylene))bis(oxy))diphthalonitrile monomer of formula (1) under conditions effective to form the thermoset polymer, wherein R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or phenyl; each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl; and p and q are each independently 0 to 4, preferably 0 or 1, more preferably 0.

Aspect 13. The method of aspect 12, wherein the monomer is a 4,4'-(((3-oxo-2-arylisoindoline-1,1-diyl)bis(4,1-phenylene))bis(oxy))diphthalonitrile monomer of formula (1a) wherein each occurrence of R¹ is independently a phenyl or a C₁₋₆ alkyl, more preferably a C₁₋₃ alkyl; each occurrence of R² and R³ is independently a C₁₋₆ alkyl, more preferably a C₁₋₃ alkyl; and r, p, q, and s are each independently 0 or 1, preferably 0.

Aspect 14. The method of aspect 12 or 13, wherein polymerizing comprises heating the 3,3-bis(3,4-dicyanophenyloxyaryl)phthalimidine at a temperature of 200-500°C, preferably 200-400°C, more preferably 200-300°C, even more preferably 220-300°C at a pressure of 1-100 atmospheres (101.3 to 10132.5 kilopascals (kPa)), preferably 1-10 atmospheres (101.3 to 1013.3 kPa), more preferably 1-5 atmospheres (101.3 to 506.7 kPa) in the presence of a curing agent.

Aspect 15. The method of aspect 14, wherein the curing agent comprises a diamine, a triamine, a higher amine, an acid salt of an amine, or an organic acid having a pKa of less than 4 in water; preferably an amine-containing phosphazene, an aromatic diamine, an aromatic diamine/metal salt complex, an aromatic ether amine, an amine salt of p-toluene sulfonic acid, or an organic acid having a pKa of less than 3 in water; more preferably an aromatic diamine or an organic acid having a pKa of less than 2 in water.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

All ranges disclosed herein are inclusive of the endpoints, and the endpoints are independently combinable with each other (e.g., ranges of "up to 25 wt%, or, more specifically, 5 wt% to 20 wt%", is inclusive of the endpoints and all intermediate values of the ranges of "5 wt% to 25 wt%," etc.). "Combinations" is inclusive of blends, mixtures, alloys, reaction products, and the like. The terms "a" and "an" and "the" do not denote a limitation of quantity, and are to be construed to cover both the singular and the plural, unless otherwise indicated herein or clearly contradicted by context. "Or" means "and/or" unless clearly stated otherwise. Reference throughout the specification to "some embodiments", "an embodiment", "an example" and so forth, means that a particular element described in connection with the embodiment or example is included in at least one embodiment or example described herein, and may or may not be present in other embodiments or examples. In addition, it is to be understood that the described elements can be combined in any suitable manner in the various embodiments. "Combination thereof' is an open term that includes one or more of the named elements, optionally together with a like element not named.

The term "hydrocarbyl" and "hydrocarbon" refers broadly to a group comprising carbon and hydrogen, optionally with 1 to 3 heteroatoms, for example, oxygen, nitrogen, halogen, silicon, sulfur, or a combination thereof. The term "aliphatic" means a branched or unbranched, saturated or unsaturated group containing carbon and hydrogen, optionally with 1 to 3 heteroatoms, for example, oxygen, nitrogen, halogen, silicon, sulfur, or a combination thereof. The term "cycloaliphatic" means a saturated or unsaturated group comprising carbon and hydrogen optionally with 1 to 3 heteroatoms, for example, oxygen, nitrogen, halogen, silicon, sulfur, or a combination thereof. The term "alkyl" means a branched or straight chain, unsaturated aliphatic hydrocarbon group, e.g., methyl, ethyl, n-propyl, i-propyl, n-butyl, s-butyl, t-butyl, n-pentyl, s-pentyl, and n- and s-hexyl. "Alkenyl" means a straight or branched chain, monovalent hydrocarbon group having at least one carbon-carbon double bond (e.g., ethenyl (-HC=CH₂)). "Alkoxy" means an alkyl group that is linked via an oxygen (i.e., alkyl-O-), for example methoxy, ethoxy, and sec-butyloxy groups. "Alkylene" means a straight or branched chain, saturated, divalent aliphatic hydrocarbon group (e.g., methylene (-CH₂-) or, propylene (-(CH₂)₃-)). "Cycloalkylene" means a divalent cyclic alkylene group, -CₙH₂ₙ₋ₓ, wherein x is the number of hydrogens replaced by cyclization(s). "Cycloalkenyl" means a monovalent group having one or more rings and one or more carbon-carbon double bonds in the ring, wherein all ring members are carbon (e.g., cyclopentyl and cyclohexyl). "Aryl" means an aromatic hydrocarbon group containing the specified number of carbon atoms, such as phenyl, tropone, indanyl, or naphthyl. "Arylene" means a divalent aryl group. "Alkylarylene" means an arylene group substituted with an alkyl group. "Arylalkylene" means an alkylene group substituted with an aryl group (e.g., benzyl). The prefix "halo" means a group or compound including one more of a fluoro, chloro, bromo, or iodo substituent. A combination of different halo groups (e.g., bromo and fluoro), or only chloro groups can be present. The prefix "hetero" means that the compound or group includes at least one ring member that is a heteroatom (e.g., 1, 2, or 3 heteroatom(s)), wherein the heteroatom(s) is each independently N, O, S, Si, or P.

Unless otherwise indicated, each of the foregoing groups can be unsubstituted or substituted, provided that the substitution does not significantly adversely affect synthesis, stability, or use of the compound. "Substituted" means that the compound, group, or atom is substituted with at least one (e.g., 1, 2, 3, or 4) substituents instead of hydrogen, where each substituent is independently nitro (-NO₂), cyano (-CN), hydroxy (-OH), halogen, thiol (-SH), thiocyano (-SCN), C₁₋₆ alkyl, C₂₋₆ alkenyl, C₂₋₆ alkynyl, C₁₋₆ haloalkyl, C₁₋₉ alkoxy, C₁₋₆ haloalkoxy, C₃₋₁₂ cycloalkyl, C₅₋₁₈ cycloalkenyl, C₆₋₁₂ aryl, C₇₋₁₃ arylalkylene (e.g., benzyl), C₇₋₁₂ alkylarylene (e.g., toluyl), C₄₋₁₂ heterocycloalkyl, C₃₋₁₂ heteroaryl, C₁₋₆ alkyl sulfonyl (-S(=O)₂-alkyl), C₆₋₁₂ arylsulfonyl (-S(=O)₂-aryl), or tosyl (CH₃C₆H₄SO₂-), provided that the substituted atom's normal valence is not exceeded, and that the substitution does not significantly adversely affect the manufacture, stability, or desired property of the compound. When a compound is substituted, the indicated number of carbon atoms is the total number of carbon atoms in the compound or group, including those of any substituents.

Unless specified to the contrary herein, all test standards are the most recent standard in effect as of the filing date of this application, or, if priority is claimed, the filing date of the earliest priority application in which the test standard appears. Unless defined otherwise, technical and scientific terms used herein have the same meaning as is commonly understood by one of skill in the art to which this application belongs. All cited patents, patent applications, and other references are incorporated herein by reference in their entirety. However, if a term in the present application contradicts or conflicts with a term in the incorporated reference, the term from the present application takes precedence over the conflicting term from the incorporated reference.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A thermoset polymer product comprising 4,4'-(((3-oxo-isoindoline-1,1-diyl)bis(4,1-arylene))bis(oxy))dibenzonitrile units of formula (2) 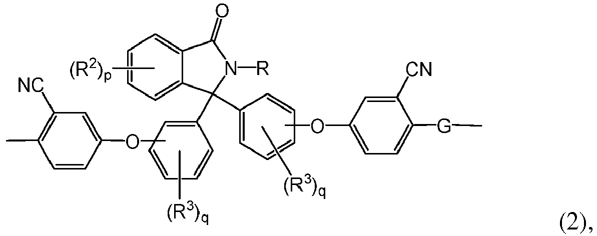 wherein the units are derived from a 4,4'-(((3-oxo-isoindoline-1,1-diyl)bis(4,1-arylene))bis(oxy))diphthalonitrile monomer of formula (1) 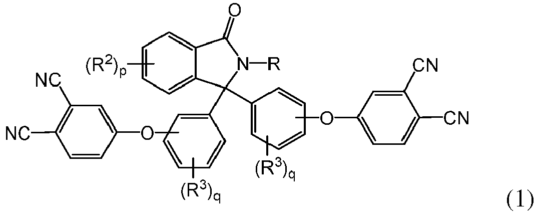 wherein
R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl;
each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl;
p and q are each independently 0 to 4, preferably 0 or 1, more preferably 0; and
G is a residue of a cyano group, and is a C₁₋₂₅ hydrocarbyl linking group having a valence of at least 2.

2. The thermoset polymer product of claim 1, wherein the monomer is a 4,4'-(((3-oxo-2-arylisoindoline-1,1-diyl)bis(4,1-phenylene))bis(oxy))diphthalonitrile monomer of formula (1a) 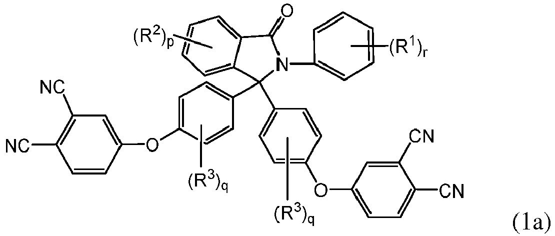 wherein
each occurrence of R¹ is independently a hydrogen, a phenyl, or a C₁₋₆ alkyl, preferably a hydrogen or a C₁₋₃ alkyl;
each occurrence of R² and R³ is independently a hydrogen or a C₁₋₆ alkyl, preferably a hydrogen or a C₁₋₃ alkyl; and
r, p, and q are each independently 0 or 1, preferably 0.

3. The thermoset polymer product of claim 2, wherein the monomer is 4'-(((3-oxo-2-phenylisoindoline-1,1-diyl)bis(4,1-phenylene))bis(oxy))diphthalonitrile of formula (1b)

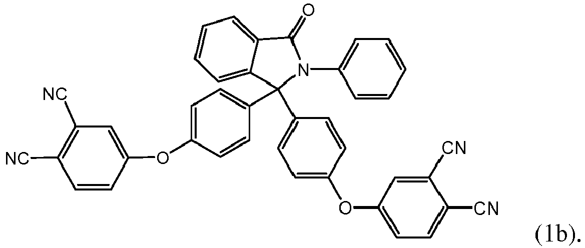

4. The thermoset polymer product of any one or more of the preceding claims, comprising 4,4'-(((3-oxo-2-arylisoindoline-1,1-diyl)bis(4,1-phenylene))bis(oxy))dibenzonitrile units of formula (2a) 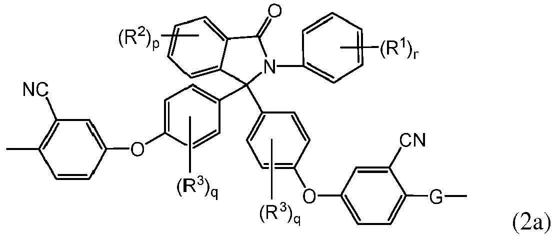 wherein
each occurrence of R¹ is independently a hydrogen, phenyl, or C₁₋₆ alkyl, preferably a hydrogen or C₁₋₃ alkyl;
each occurrence of R² and R³ is independently a hydrogen or C₁₋₆ alkyl, preferably a hydrogen or C₁₋₃ alkyl;
r, p, and q are each independently 0 or 1, preferably 0; and
G is a residue of a nitrile group, wherein G is a C₁₋₆ hydrocarbyl linking group having a valence of 2-4.

5. The thermoset polymer product of any one or more of the preceding claims, wherein G is a phthalocyanine group or a triazinyl group.

6. The thermoset polymer product of any one or more of the preceding claims, having the formula (2c)

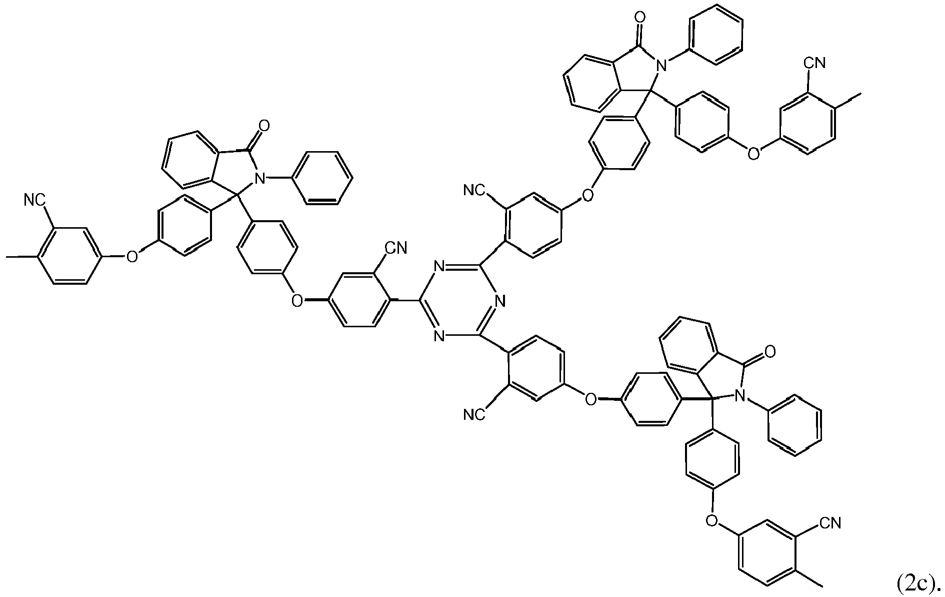

7. The thermoset polymer product of any one or more of the preceding claims, comprising 4,4'-(((3-oxo-isoindoline-1,1-diyl)bis(4,1-arylene))bis(oxy))dibenzo units of formula (3) 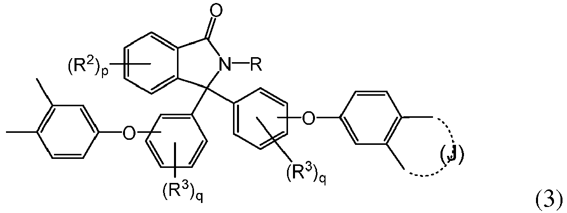 wherein
R is a C₁₋₂₅ hydrocarbyl, preferably a C₁₋₆ alkyl, phenyl, or phenyl substituted with up to five C₁₋₆ alkyl groups, more preferably a C₁₋₃ alkyl or phenyl;
each occurrence of R² and R³ is independently a hydrogen, halogen, or C₁₋₂₅ hydrocarbyl, preferably a hydrogen, halogen, or C₁₋₆ alkyl, more preferably a hydrogen or C₁₋₃ alkyl; and
p and q are each independently 0 to 4, preferably 0 or 1, more preferably 0; and
J is a residue of a cyano group, and is a C₁₋₂₅ hydrocarbyl linking group having a valence of at least 3.

8. The thermoset polymer product of claim 7, comprising 4,4'-(((3-oxo-2-arylisoindoline-1,1-diyl)bis(4,1-phenylene))bis(oxy))dibenzo units of formula (3a) 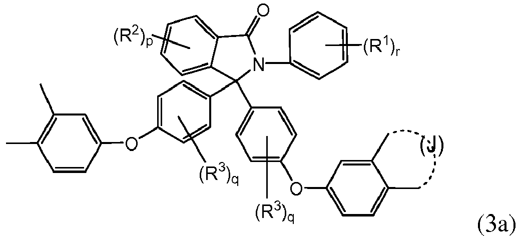 wherein
each occurrence of R¹ is independently a hydrogen, phenyl, or C₁₋₆ alkyl, preferably a hydrogen or C₁₋₃ alkyl;
each occurrence of R² and R³ is independently a hydrogen or C₁₋₆ alkyl, preferably a hydrogen or C₁₋₃ alkyl;
r, p, and q are each independently 0 or 1, preferably 0; and
J is a residue of two nitrile groups, wherein J is a C₄₋₁₀ hydrocarbyl linking group having a valence of 2 to 4.

9. The thermoset polymer product of claim 7 or 8, having the formula (3c)

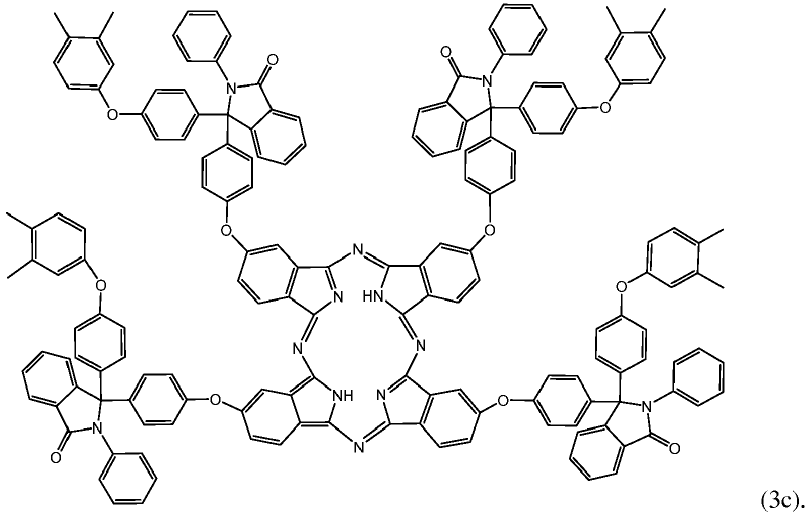

10. A polymer composition comprising
the thermoset polymer product of any one or more of the preceding claims, and
an additive, preferably wherein the additive is a particulate filler, a fibrous filler, an antioxidant, a heat stabilizer, a light stabilizer, a ultraviolet light stabilizer, a ultraviolet light-absorbing compound, a near infrared light-absorbing compound, a infrared light-absorbing compound, a plasticizer, a lubricant, a release agent, a antistatic agent, an anti-fog agent, a antimicrobial agent, a colorant, a surface effect additive, a radiation stabilizer, a flame retardant, an anti-drip agent, a fragrance, a polymer different from the thermoset polymer, or a combination thereof.

11. An article comprising the polymer composition of claim 10, wherein the article is a composite, a foam, a fiber, a layer, a coating, an encapsulant, an adhesive, a sealant, a component, a prepreg, a casing, or a combination thereof;
preferably wherein the article is an electronic substrate, an electronic housing, a microelectronic device, a printed electronic component, a tooling, a geothermal tool, an oil rig component, a flame retardant component, a sizing resin, a resin infusion molded component, a resin transfer molded component, or a combination thereof.

12. A method for the manufacture of the thermoset polymer product of any one or more of claims 1 or 9, the method comprising
polymerizing a 4,4'-(((3-oxo-isoindoline-1,1-diyl)bis(4,1-arylene))bis(oxy))diphthalonitrile monomer of formula (1) 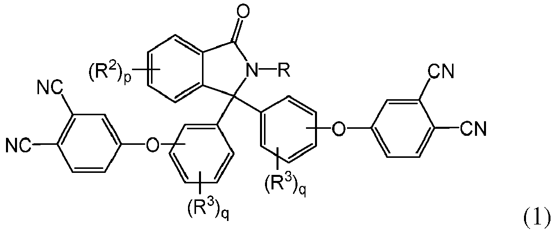 under conditions effective to form the thermoset polymer, wherein
R is a C₁₋₂₅ hydrocarbyl, preferably a C₁₋₆ alkyl, a phenyl, or a phenyl substituted with up to five C₁₋₆ alkyl groups, more preferably a C₁₋₃ alkyl or phenyl;
each occurrence of R² and R³ is independently a hydrogen, a halogen, or a C₁₋₂₅ hydrocarbyl, preferably a hydrogen, a halogen, or a C₁₋₆ alkyl, more preferably a hydrogen or a C₁₋₃ alkyl; and
p and q are each independently 0 to 4, preferably 0 or 1, more preferably 0.

13. The method of claim 12, wherein the monomer is a 4,4'-(((3-oxo-2-arylisoindoline-1,1-diyl)bis(4,1-phenylene))bis(oxy))diphthalonitrile monomer of formula (1a) 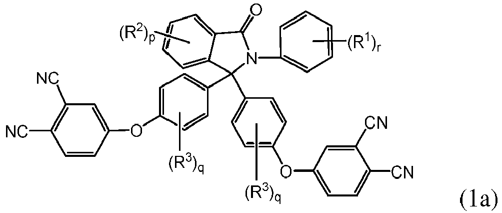 wherein
each occurrence of R¹ is independently a phenyl or a C₁₋₆ alkyl, more preferably a C₁₋₃ alkyl;
each occurrence of R² and R³ is independently a C₁₋₆ alkyl, more preferably a C₁₋₃ alkyl; and
r, p, q, and s are each independently 0 or 1, preferably 0.

14. The method of claim 12 or 13, wherein polymerizing comprises heating the 3,3-bis(3,4-dicyanophenyloxyaryl)phthalimidine at a temperature of 200 to 500°C, preferably 200 to 400°C, more preferably 200 to 300°C, even more preferably 220 to 300°C at a pressure of 101.3 to 10132.5 kPa, preferably 101.3 to 1013.3 kPa, more preferably 101.3 to 506.7 kPa, preferably in the presence of a curing agent.

15. The method of claim 14, wherein the curing agent comprises a diamine, a triamine, a higher amine, an acid salt of an amine, or an organic acid having a pKa of less than 4 in water;
preferably an amine-containing phosphazene, an aromatic diamine, an aromatic diamine/metal salt complex, an aromatic ether amine, an amine salt of p-toluene sulfonic acid, or an organic acid having a pKa of less than 3 in water;
more preferably an aromatic diamine or an organic acid having a pKa of less than 2 in water.
